# EUROPÄISCHE PATENTSCHRIFT

(11) **EP 4 093 374 B1**
(45) Veröffentlichungstag und Bekanntmachung des Hinweises auf die Patenterteilung: **15.11.2023**
(21) Anmeldenummer: 21701961.1
(22) Anmeldetag: 22.01.2021
(51) Int. Cl.: A61K 9/00, A61K 9/14, A61K 9/16, A61K 31/352, A61K 9/08, A61K 47/32

(54) **KOMPLEX AUS 7-DEACETYLFORSKOLIN UND PVP**
COMPLEX OF 7-DEACETYLFORSKOLIN AND PVP
COMPLEXE DES 7-DÉACÉTYLFORSKOLINE ET PVP

(30) Priorität: 23.01.2020 EP 20153429
(43) Veröffentlichungstag der Anmeldung: 30.11.2022
(73) Patentinhaber: SciPharm S.à r.l., 6689 Mertert (LU)
(72) Erfinder: KUBIN, Andreas, 3032 Eichgraben (AT)
(74) Vertreter: Loidl, Manuela Bettina
(86) Internationale Anmeldenummer: PCT/EP2021/051499
(87) Internationale Veröffentlichungsnummer: WO 2021/148633

(56) Entgegenhaltungen:
- WO-A1-2017/103840
- WO-A2-2005/025500
- US-B1- 6 346 273
- K. Kolter ET AL: "Hot-Melt Extrusion with BASF Pharma Polymers" In: "Hot-Melt Extrusion with BASF Pharma Polymers", 1. Oktober 2012 (2012-10-01), XP055242593, ISBN: 978-3-00-039415-7 in der Anmeldung erwähnt das ganze Dokument Seite 88 - Seite 91

## Beschreibung

### GEBIET DER ERFINDUNG

Die vorliegende Erfindung betrifft einen Komplex aus 7-Deacetylforskolin (7-DAFSK) und Polyvinylpyrrolidon (PVP) und ein Verfahren zur Herstellung eines solchen Komplexes.

### HINTERGRUND DER ERFINDUNG

Das Diterpen Forskolin ist ein nahezu wasserunlöslicher Pflanzeninhaltsstoff aus *Plectranthus barbatus* (*Coleus forskohlii*). Forskolin steht im Fokus für vielseitige medizinische Anwendungen, unter anderem bei Herz-Kreislauferkrankungen, Asthma bronchiale, Fettleibigkeit, Behandlung des Glaukoms, Lungenerkrankungen, Gelenksentzündung, und Arthrose/Arthritis. Forskolin fördert die Knorpelbildung in Gelenken und verhindert gleichzeitig die Ossifikation und Mineralisation. Weiters wird Forskolin in der Stammzellforschung im Hinblick auf Differenzierung und Rückentwicklung zur Pluripotenz untersucht.

Forskolin aktiviert die Adenylylcyclase, ein membrangebundenes Enzym. Adenylylcyclase wandelt Adenosintriphosphat (ATP) zu cyclischem Adenosinmonophosphat (cAMP) um und setzt cAMP in das Cytosol frei, wodurch die intrazelluläre Konzentration an cAMP in den meisten Zellen und Gewebearten erhöht wird (Metzger H. et al., 1981, Arzneimittelforschung, 1248-50). cAMP wirkt als Second Messenger (sekundärer Botenstoff) bei der zellulären Signaltransduktion und dient zur Aktivierung vieler Peptidhormone (Proteinkinasen).

Forskolin ist ein Diterpen mit zwei *α*-Hydroxygruppen an Position 1 und 9, einer β-Hydroxygruppe an Position 6, und einer β-Acetoxygruppe an Position 7, wobei die beiden *α*-Hydroxygruppen an Position 1 und 9 essentiell für die Bindung an die Adenylylcyclase sind. Forskolin braucht aufgrund seiner geringen Wasserlöslichkeit (0,01 mg/mL, WO2005025500A2) verschiedene Formulierungen, um als Diagnostikum oder Therapeutikum eingesetzt werden zu können. Solche Formulierungen enthalten verschiedene Lösungsmittel wie z.B. Alkohole oder DMSO, die oft nicht gut verträglich sind und zu Nebenwirkungen führen.

Das Dokument US6346273B1 behandelt eine Methode zur Erhöhung der Wasserlöslichkeit von schwer wasserlöslichen pharmazeutisch aktiven Substanzen, wie z.B. Forskolin, durch Komplexierung mit einem nichtionischen Polymer, wie z.B. Polyvinylpyrrolidon (PVP). In diesem Dokument wird eine Erhöhung der Wasserlöslichkeit von Forskolin beschrieben.

Eine erhöhte Wasserlöslichkeit von Forskolin wird durch Komplexierung von Forskolin mit Cyclodextrinen erreicht. Die WO2005025500A2 beschreibt eine Methode zur Komplexierung von Forskolin mit Cyclodextrin im wässrigen Medium.

WO2017103840A1 beschreibt wasserlösliche Forskolin-Zusammensetzungen durch Komplexierung mit wasserlöslichen Polymeren wie u.a. PVP. Nach Mischung von Forskolin mit dem Polymer wird eine Stärkelösung zugegeben und die Mischung erhitzt, dann abgekühlt und sprühgetrocknet.

Die WO2018127600A1 offenbart eine Methode zur Herstellung von Forskolin-Cyclodextrin-Komplexen, welche auf einem Sinterprozess beruht und einen erhöhten Gehalt von Forskolin im Komplex, sowie eine erhöhte Wasserlöslichkeit ermöglicht.

Ein anderer Ansatz, um die Wasserlöslichkeit von Forskolin zu erhöhen, ist eine chemische Derivatisierung. Ein Forskolinderivat mit höherer Wasserlöslichkeit im Vergleich zu Forskolin ist 7-Deacetyl Forskolin, welches eine ähnliche Wirkung auf die Adenylylcyclase zeigt wie Forskolin (Laurenza A. et al, Molecular Pharmacology 1987, 32(1), 133-139, Pinto C. et al, Biochemical Pharmacology 2009, 78(1), 62-69). Die Deacetylierung von Forskolin an Position 7 kann die Wasserlöslichkeit jedoch nur bedingt erhöhen (bis 1 mg/mL).

Daher besteht der Bedarf an einer verbesserten wasserlöslichen Form eines Forskolinderivats, welche sowohl durch hohe Wasserlöslichkeit und damit verbesserte Bioverfügbarkeit, als auch durch einen hohen Gehalt an Forskolinderivat gekennzeichnet ist.

Eine Aufgabe der vorliegenden Erfindung ist es daher, eine Form von 7-Deacetyl Forskolin mit erhöhter Wasserlöslichkeit und gleichzeitig hohem Gehalt an 7-Deacetyl Forskolin zur Verfügung zu stellen.

### ZUSAMMENFASSUNG DER ERFINDUNG

Gelöst wird diese Aufgabe durch einen Komplex aus 7-Deacetylforskolin (7-DAFSK) und Polyvinylpyrrolidon (PVP), wie in den Ansprüchen und Ausführungsformen der vorliegenden Erfindung beschrieben.

Die vorliegende Erfindung offenbart einen Komplex aus 7-Deacetylforskolin (7-DAFSK) und Polyvinylpyrrolidon (PVP).

In einer Ausführungsform ist der erfindungsgemäße Komplex dadurch gekennzeichnet, dass der mittlere Masseanteil von 7-DAFSK am Gesamtkomplex im Bereich von 1 bis 50 Gew.-% liegt, bevorzugt bei 5, 10, 15, 20 oder 25 Gew.-%, besonders bevorzugt bei 30 Gew.-%, 35 Gew.-%, 40 Gew.-%, 45 Gew.-%, oder höher.

In einer Ausführungsform ist das mittlere Stoffmengenverhältnis von 7-DAFSK zu PVP im Komplex 0,1 oder höher, bevorzugt 1, 2, 3, 4, 5, 6, 7, 8, 9, 10, 20, 30, 40, 50, 60 oder höher, besonders bevorzugt 3 oder höher.

In einer Ausführungsform ist der erfindungsgemäße Komplex dadurch gekennzeichnet, dass PVP eine mittlere molare Masse von 1 bis 40 kD hat. Vorzugsweise hat das PVP eine mittlere molare Masse von 1 bis 25 kD, besonders bevorzugt etwa 2 bis 3 kD, ganz besonders bevorzugt etwa 2,5 kD.

In einer weiteren Ausführungsform ist der erfindungsgemäße Komplex dadurch gekennzeichnet, dass 7-DAFSK von synthetischem Forskolin stammt.

In einer Ausführungsform liegt das 7-DAFSK in einer Reinheit von mindestens 98,5 % vor.

Es wurde überraschend gefunden, dass ein besonders hoher Masseanteil an 7-DAFSK am 7-DAFSK-PVP Komplex dadurch erreicht werden kann, dass ein Gemisch von 7-DAFSK und PVP auf eine Temperatur erhitzt wird, die über der Glasübergangstemperatur des eingesetzten PVP liegt. Insbesondere wird das Gemisch auf eine Temperatur erhitzt, welche mindestens 20°C, bevorzugt mindestens 25°C, 30°C, 40°C, 50°C, 60°C, 70°C, 80°C, 90°C, 100°C über der Glasübergangstemperatur des PVP liegt. Insbesondere wird die Temperatur auf einen Bereich von etwa 25 bis 50°C über der Glasübergangstemperatur des PVP gebracht.

Die vorliegende Erfindung stellt daher auch ein Verfahren zur Herstellung des erfindungsgemäßen 7-DAFSK-PVP Komplexes zur Verfügung, welches dadurch gekennzeichnet ist, dass ein Gemisch von 7-DAFSK und PVP auf eine Temperatur erhitzt wird, die über der Glasübergangstemperatur des eingesetzten PVP liegt.

In einer Ausführungsform ist das erfindungsgemäße Verfahren dadurch gekennzeichnet, dass dem Gemisch von 7-DAFSK und PVP ein Lösungsmittel, vorzugsweise Wasser, Ethanol, Methanol, Aceton, Ethylmethylketon, Dichlormethan und/oder Ethylacetat, noch mehr bevorzugt Wasser, Ethanol, Methanol, Propanol und/oder Dichlormethan, zugegeben wird. Besonders bevorzugt wird dem Gemisch von 7-DAFSK und PVP Wasser und/oder Ethanol, oder ein Gemisch davon, zugegeben.

In einer speziellen Ausführungsform wird das erfindungsgemäße Gemisch für mindestens 5 Minuten auf einer Temperatur gehalten, die über der Glasübergangstemperatur des eingesetzten PVP liegt.

In einer Ausführungsform enthält das erfindungsgemäße Verfahren folgende Schritte:
a) Herstellung einer Mischung aus 7-DAFSK und PVP und Versetzen der Mischung mit einem Lösungsmittel, bevorzugt mit einem Alkohol, bevorzugt Ethanol,
b) Zugabe von Wasser,
c) Erhitzen der Mischung, über die Glasübergangstemperatur des PVPs für mindestens 5 Minuten,
d) Abkühlen der Mischung und Lösen in Wasser,
e) Entfernen der ungelösten Bestandteile, optional durch Filtern.
In einer speziellen Anwendungsform wird die Mischung mindestens 20, 25, 30, 35, 40, 45, 50, 55, 60°C, 70°C, 80°C, 90°C, 100°C über die Glasübergangstemperatur des PVPs erhitzt.

Die Anwendung des erfindungsgemäßen Verfahrens erlaubt die effiziente und unkomplizierte Herstellung von 7-DAFSK-PVP Komplexen, welche die Wasserlöslichkeit von 7-DAFSK deutlich erhöhen und auch einen hohen Masseanteil von 7-DAFSK enthalten können, was vorteilhafte Anwendungen von 7-DAFSK in Diagnose und/oder Therapie ermöglicht.

Mit den erfindungsgemäßen Komplexen von 7-DAFSK mit PVP kann die Wasserlöslichkeit von 7-DAFSK erhöht werden. Durch einen hohen Masseanteil von 7-DAFSK am erfindungsgemäßen Komplex kann die Wasserlöslichkeit von 7-DAFSK erhöht werden, ohne dass damit eine unvorteilhaft große Menge an PVP benötigt wird. Formulierungen mit Forskolinderivaten können damit auch materialsparender als bisher zusammengestellt werden. Dadurch, dass große Mengen an PVP und damit einhergehende Nebenwirkungen vermieden werden können, erleichtert und verbessert die vorliegende Erfindung die Anwendung von 7-DAFSK in Diagnose und/oder Therapie.

Die vorliegende Erfindung stellt daher auch eine pharmazeutische Zusammensetzung enthaltend einen erfindungsgemäßen 7-DAFSK-PVP Komplex zur Verfügung.

In einer Ausführungsform ist besagte pharmazeutische Zusammensetzung dadurch gekennzeichnet, dass die Zusammensetzung 7-DAFSK in einer Konzentration von mindestens 25 mg/L, 50 mg/L, 75 mg/L, 100 mg/L, 200 mg/L, 300 mg/L, 400 mg/L, 500 mg/L, 600 mg/L, 800 mg/L, 1000 mg/L, 2000 mg/L, 3000 mg/L, oder 4000 mg/L enthält, bevorzugt 4000 mg/L.

Ein Aspekt der vorliegenden Erfindung betrifft eine pharmazeutische Zusammensetzung zur oralen, intravenösen oder inhalativen Verabreichung.

In einer weiteren speziellen Ausführungsform wird die erfindungsgemäße pharmazeutische Zusammensetzung zur Behandlung von Herz-Kreislauferkrankungen, Asthma bronchiale, Fettleibigkeit, Glaukom, Lungenerkrankungen, Gelenksentzündung, oder Arthrose/Arthritis verwendet.

Ein Aspekt der vorliegenden Erfindung betrifft eine erfindungsgemäße pharmazeutischen Zusammensetzung zur Verwendung als Medikament.

### FIGUREN

**Figur 1****:** Reaktionsschema zur Deacetylierung von Forskolin zu 7-DAFSK durch Natriumcarbonat (Na₂CO₃).
**Figur 2****:** Vergleich des Masseanteils von 7-DAFSK an 7-DAFSK-PVP K12 und 7-DAFSK-PVP K25-Komplexen in mittels Erhitzungsverfahren und Lösungsverfahren hergestellten Proben.
**Figur 3****:** Anteil an umgesetztem 7-DAFSK bei verschiedenen eingesetzten Gewichtsverhältnissen von DAFSK zu PVP K12.
**Figur 4****:** Vergleich des Masseanteils von 7-DAFSK an 7-DAFSK-PVP K12 Komplexen bei unterschiedlichen Gewichtsverhältnissen von 7-DAFSK zu PVP K12.
**Figur 5****:** Zusammenhang zwischen der Wasserlöslichkeit und dem Masseanteil von 7-DAFSK am 7-DAFSK-PVP K12 Komplex.
**Figur 6****:** cAMP Assay mit HEK293 Zellen, welche EP2, EP4 und IP Rezeptoren enthalten. Die Zellen wurden mit einer Kontrollprobe (Treprostinil), 30µM Forskolin in DMSO, 100 µM 7-DAFSK in Wasser und 100 µM 7-DAFSK im 7-DAFSK-PVP K12 Komplex in Wasser stimuliert.

### DETAILLIERTE BESCHREIBUNG DER ERFINDUNG

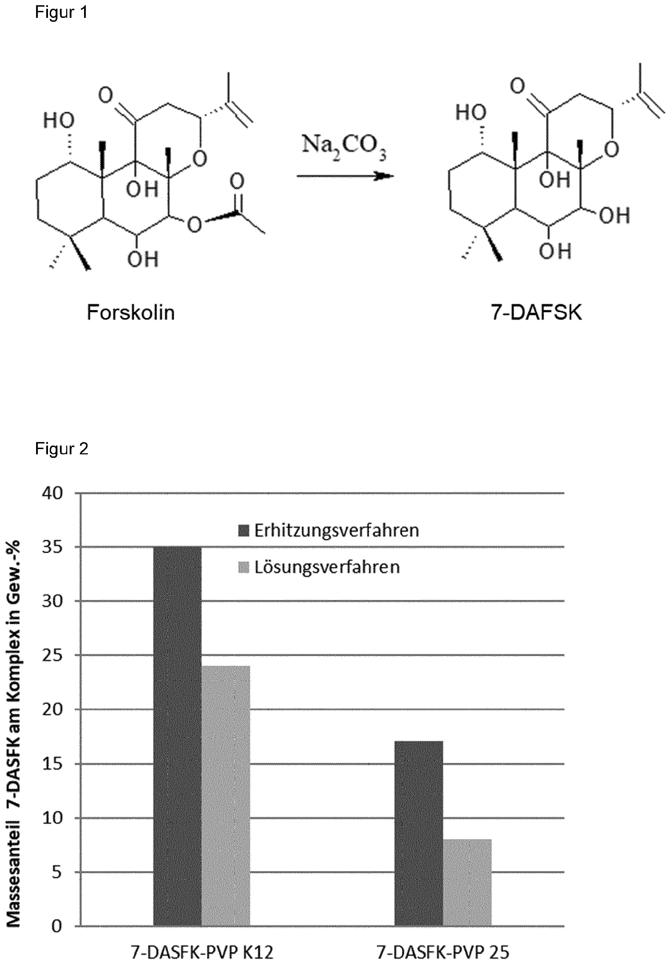

7-Deacetylforskolin (7-DAFSK) kann durch die in Figur 1 gezeigte Strukturformel dargestellt werden. 7-DAFSK kann neben der molekularen Form auch in anderen Formen vorliegen, wie in der Form von Salzen, z.B. Alkalimetallsalzen wie Natrium- oder Kaliumsalzen. Der Begriff "7-Deacetylforskolin (7-DAFSK)" bezeichnet im Rahmen der vorliegenden Erfindung sowohl die molekulare Form, wie in der Strukturformel in Figur 1 dargestellt, als auch alle anderen möglichen Formen, wie Salze von 7-DAFSK, vorzugsweise Alkalisalze, besonders bevorzugt ein Natrium- oder Kaliumsalz.

Polyvinylpyrrolidon (PVP), auch Polyvidon oder Povidon, ist ein Polymer der Verbindung Vinylpyrrolidon. Handelsüblich ist PVP in unterschiedlichen Polymerisationsgraden erhältlich. Der Polymerisierungsgrad bestimmt die mittlere molare Masse des Polymers. Der Begriff "mittlere molare Masse" bezieht sich hierin auf das Massenmittel der Molmasse.

Im Rahmen der vorliegenden Erfindung wird PVP mit einer mittleren molaren Masse von 1 bis 40 kD bevorzugt, weil Polymere in dieser Größe noch gut und unmetabolisiert von der Niere ausgeschieden werden können.

In einer Ausführungsform ist der 7-DAFSK-PVP Komplex dadurch gekennzeichnet, dass das PVP eine mittlere molare Masse von 1 bis 60 kD hat, insbesondere von 1 bis 40 kD hat. Bevorzugt ist PVP mit einer mittleren molaren Masse von 1 bis 25 kD, bevorzugt etwa 2 bis 9 kD, 2 bis 8 kD, 2 bis 7 kD, 2 bis 6 kD, 2 bis 5 kD, 2 bis 4 kD, besonders bevorzugt 2 bis 3 kD, ganz besonders bevorzugt etwa 2,5 kD.

Der Begriff "etwa" bezieht sich hierin auf den angegebenen Wert, oder einen Wert, welcher von dem angegebenen Wert um +/-10 % abweicht.

Das Molekulargewicht von PVP kann anstelle der Angabe des Massenmittels der Molmasse auch über den K-Wert definiert werden, welcher durch Viskositätsmessung erhalten wird und vom Viskositätsmittel der Molmasse abhängt. PVP mit einem Massenmittel der Molmasse von etwa 2,5 kD entspricht PVP mit einem K-Wert von etwa 12, hier als "PVP K12" bezeichnet (K. Kolter et al. "Hot-Meld Extrusion with BASF Pharma Polymers", ISBN 978-3-00-039415-7, 2012). PVP mit einem Massenmittel der Molmasse von etwa 24 kD entspricht PVP mit einem K-Wert von etwa 25, hier als "PVP K25" bezeichnet.

Der Begriff "7-DAFSK-PVP Komplex" bezeichnet im Rahmen der vorliegenden Erfindung ein chemisches Erzeugnis, welches 7-DAFSK und PVP enthält und dadurch gekennzeichnet ist, dass eine Verbindung zwischen 7-DAFSK und PVP besteht. Der Begriff "Komplex" schränkt die Art der Verbindung in keiner Weise ein, sondern bedeutet lediglich, dass eine Verbindung zwischen einem oder mehreren 7-DAFSK Molekülen und einem oder mehreren PVP Molekülen besteht. Die Verbindung kann z.B. eine kovalente Bindung oder bevorzugt eine nicht-kovalente Anlagerung von 7-DAFSK an PVP sein, z.B. durch Van-der-Waals-Bindungen oder Wasserstoffbrückenbindungen. Als "7-DAFSK-PVP" bzw. "7-DAFSK-PVP Komplex" soll das chemische Erzeugnis insgesamt und nicht ein einzelner 7-DAFSK-PVP Komplex auf molekularer Ebene verstanden werden

Die Bezeichnungen "Masseanteil und "Stoffmengenverhältnis" sind hierin immer als mittlere Werte zu verstehen. Sie beziehen sich nicht auf jeden einzelnen Komplex auf molekularer Ebene, sondern auf einen mittleren Wert des gesamten chemischen Erzeugnisses.

Prozentangaben (%) beziehen sich in der vorliegenden Erfindung, wenn nicht anders angegeben, jeweils auf Gewichtsprozent (Gew.-%).

In einer Ausführungsform beträgt der mittlere Masseanteil von 7-DAFSK am Gesamtkomplex 1 bis 50 Gew.-%, bevorzugt 2, 3, 4, 5, 6, 7, 8, 9, 10, 15, 20 oder 25 Gew.-%, besonders bevorzugt 30 Gew.-%, 35 Gew.-%, 40 Gew.-%, 45 Gew.-% oder höher. Der Masseanteil gibt hier den relativen Anteil der Masse von 7-DAFSK an der Masse des Gesamtkomplexes (7-DAFSK- PVP) wieder.

Der Anteil von 7-DAFSK am 7-DAFSK-PVP Komplex kann auch als Stoffmengenverhältnis ausgedrückt werden. Beispielsweise hat ein 7-DAFSK-PVP Komplex, der aus molekularem 7-DAFSK (molare Masse: 368,5 g/mol) und PVP mit einer mittleren molaren Masse von etwa 2,5 kD ("PVP K12") besteht, und der einen mittleren Masseanteil von 7-DAFSK am Gesamtkomplex von 30 Gew.-% aufweist, ein mittleres Stoffmengenverhältnis von 7-DAFSK zu PVP im Komplex von etwa 3. Der mittlere Stoffmengenanteil gibt hier das Verhältnis der Stoffmenge von 7-DAFSK zur Stoffmenge von PVP wieder.

In einer Ausführungsform der vorliegenden Erfindung ist das mittlere Stoffmengenverhältnis von 7-DAFSK zu PVP im Komplex 0,1 oder höher, bevorzugt 3, 4, 5, 6, 7, 8, 9, 10, 15, 20, 25, 30,35, 40, 45, 50, 55, 60 oder höher, besonders bevorzugt 3 oder höher.

Der Anteil (Masseanteil wie auch Stoffmengenverhältnis) von 7-DAFSK am Gesamtkomplex ist hierin stets als mittlerer Anteil zu verstehen. Eine geeignete Methode, um den mittleren Anteil von 7-DAFSK am Gesamtkomplex zu bestimmen, ist die Hochleistungsflüssigkeitschromatographie (HPLC). Die Fachperson ist mit der Durchführung einer solchen Bestimmung vertraut.

7-DAFSK kann aus Forskolin mittels einer Deacetylierungsreaktion hergestellt werden, z.B. durch Carbonatverseifung, wie in den konkreten Beispielen der vorliegenden Erfindung erläutert wird. Als Edukt kann sowohl natürliches als auch synthethisches Forskolin verwendet werden. Natürliches Forskolin liegt typischerweise als angereichertes Extrakt vor, welches vor der Verwendung gereinigt wird. Im Rahmen der vorliegenden Erfindung wird als Edukt für die 7-DAFSK Synthese bevorzugt synthetisches Forskolin verwendet, welches in einer hohen Reinheit kommerziell erhältlich ist.

In einer Ausführungsform ist der erfindungsgemäße Komplex daher dadurch gekennzeichnet, dass 7-DAFSK von synthetischem Forskolin stammt. In einer bevorzugten Ausführungsform liegt das 7-DAFSK in einer Reinheit von mindestens 98,5 % vor.

Der erfindungsgemäße 7-DAFSK-PVP Komplex ist durch eine erhöhte Wasserlöslichkeit im Vergleich zu nicht komplexiertem 7-DAFSK-PVP gekennzeichnet, was für pharmazeutische Anwendungen vorteilhaft ist.

Es wurde überraschend gefunden, dass 7-DAFSK-PVP Komplexe synthetisch leicht zugänglich sind, indem 7-DAFSK und PVP auf eine Temperatur erhitzt werden, die über der Glasübergangstemperatur des eingesetzten PVP liegt. Überraschenderweise führt dieses Verfahren auch zu einem hohen Masseanteil an 7-DAFSK im Gesamtkomplex.

Die vorliegende Erfindung umfasst daher ein Verfahren zur Herstellung eines erfindungsgemäßen 7-DAFSK-PVP Komplexes, dadurch gekennzeichnet, dass ein Gemisch von 7-DAFSK und PVP auf eine Temperatur erhitzt wird, die über der Glasübergangstemperatur des eingesetzten PVP liegt.

PVP hat als amorphe Substanz keinen Schmelzpunkt, sondern zeigt eine sogenannte Glasübergangstemperatur. Die Glasübergangstemperatur ist unter anderem vom Polymerisierungsgrad, also von der mittleren molaren Masse, von PVP abhängig (siehe Tabelle 1).

**Tabelle 1: Beispiele für die Glasübergangstemperatur von PVP in Abhängigkeit der molaren Masse.**

| **Mittlere molare Masse von PVP** | **Glasübergangstemperatur** |
|---|---|
| etwa 2.5 kD ("PVP K12") | 93 °C |
| etwa 9 kD ("PVP K17") | 130 °C |
| etwa 24 kD ("PVP K25") | 155 °C |
| etwa 40 kD ("PVP K30") | 175 °C |

Als "Glasübergangstemperatur des eingesetzten PVPs" soll in diesem Zusammenhang die Temperatur verstanden werden, bei der der Glasübergang von PVP im vorliegenden Gemisch mit 7-DAFSK erfolgt. Die Glasübergangstemperatur des eingesetzten PVPs kann von der Zusammensetzung des Gemisches beeinflusst werden, beispielsweise wenn der Mischung Lösungsmittel oder Wasser zugesetzt werden. Methoden zur Bestimmung der Glasübergangstemperatur sind der Fachperson bekannt. Vorzugsweise kann die Glasübergangstemperatur dem Verfahren der entsprechenden DIN-Norm folgend bestimmt werden (Wampfler B., et al., Messunsicherheit in der Kunststoffanalytik - Ermittlung mit Ringversuchsdaten. Carl Hanser Verlag, München 2017, ISBN-10 : 3446452869).

Eine besonders bevorzugte Ausführungsform des erfindungsgemäßen Verfahrens zur Herstellung des 7-DAFSK-PVP Komplexes ist dadurch gekennzeichnet, dass die mittlere molare Masse des eingesetzten PVP bei etwa 2,5 kD ("PVP K12") liegt und die Mischung auf eine Temperatur von über 93°C erhitzt wird. Im Speziellen wird auf eine Temperatur erhitzt, die mindestens 25°C, insbesondere 30°C, 40°C, 45°C oder mehr über der Glasübergangstemperatur des PVP K12 liegt.

Eine weitere bevorzugte Ausführungsform ist dadurch gekennzeichnet, dass die mittlere molare Masse des eingesetzten PVP bei etwa 9 kD ("PVP K17") liegt und die Mischung auf eine Temperatur von über 130°C erhitzt wird. Im Speziellen wird auf eine Temperatur erhitzt, die mindestens 25°C, insbesondere 30°C, 40°C, 45°C oder mehr über der Glasübergangstemperatur des PVP K17 liegt.

Eine weitere bevorzugte Ausführungsform ist dadurch gekennzeichnet, dass die mittlere molare Masse des eingesetzten PVP bei etwa 24 kD ("PVP K 25") liegt und die Mischung auf eine Temperatur von über 155°C erhitzt wird. Im Speziellen wird auf eine Temperatur erhitzt, die mindestens 25°C, insbesondere 30°C, 40°C, 45°C oder mehr über der Glasübergangstemperatur des PVP K 25 liegt.

Eine weitere bevorzugte Ausführungsform ist dadurch gekennzeichnet, dass die mittlere molare Masse des eingesetzten PVP bei etwa 40 kD ("PVP K 30") liegt und die Mischung auf eine Temperatur von über 175°C erhitzt wird. Im Speziellen wird auf eine Temperatur erhitzt, die mindestens 25°C, insbesondere 30°C, 40°C, 45°C oder mehr über der Glasübergangstemperatur des PVP K 30 liegt.

Es hat sich als vorteilhaft erwiesen, dem erfindungsgemäßen Gemisch von 7-DAFSK und PVP ein Lösungsmittel oder ein Gemisch von Lösungsmitteln zuzufügen. Vorzugsweise wird das Gemisch von 7-DAFSK und PVP in wenig Lösungsmittel pastös verrührt. Die Zugabe von Lösungsmittel kann dazu beitragen, dass sich 7-DAFSK und PVP homogen verteilen und sich größere Mengen an 7-DAFSK an PVP anlagern können. Als Lösungsmittel eignen sich wässrige, alkoholische oder andere organische Lösungsmittel, wie z.B. Wasser, Ethanol, Methanol, Propanol, Aceton, Ethylmethylketon, Dichlormethan und/oder Ethylacetat.

In einer Ausführungsform ist das erfindungsgemäße Verfahren zur Herstellung des 7-DAFSK-PVP Komplexes dadurch gekennzeichnet, dass dem Gemisch von 7-DAFSK und PVP ein Lösungsmittel, vorzugsweise Wasser, Ethanol, Methanol, Propanol, Aceton, Ethylmethylketon, Dichlormethan und/oder Ethylacetat, noch mehr bevorzugt Wasser, Ethanol, Methanol, Propanol, Dichlormethan und/oder Pyridin, zugegeben wird. Besonders bevorzugt wird dem Gemisch von 7-DAFSK und PVP Wasser und/oder Ethanol, oder ein Gemisch davon zugegeben. Die Zugabe von Wasser und/oder Ethanol kann gleichzeitig oder hintereinander erfolgen.

Es hat sich im Herstellungsverfahren als zweckmäßig erwiesen, das Gemisch von 7-DAFSK und PVP für einen bestimmten Zeitraum auf einer Temperatur, die über der Glasübergangstemperatur des eingesetzten PVPs liegt, zu halten. Gute Resultate wurden erzielt, wenn die Mischung für mindestens 5 Minuten über der Glasübergangstemperatur gehalten wurde.

In einer speziellen Ausführungsform des erfindungsgemäßen Verfahrens wird das Gemisch von 7-DAFSK und PVP daher für mindestens 5 Minuten auf einer Temperatur gehalten, die über der Glasübergangstemperatur des eingesetzten PVP liegt.

In einer speziellen Ausführungsform enthält das erfindungsgemäße Verfahren folgende Schritte, welche der Reihe nach durchgeführt werden:
a) Herstellung einer Mischung aus 7-DAFSK und PVP und Versetzen der Mischung mit einem Lösungsmittel, bevorzugt mit einem Alkohol, bevorzugt Ethanol,
b) Zugabe von Wasser,
c) Erhitzen der Mischung über die Glasübergangstemperatur des PVPs für mindestens 5 Minuten,
d) Abkühlen der Mischung und Lösen in Wasser,
e) Entfernen der ungelösten Bestandteile, optional durch Filtern.

In einer Ausführungsform wird eine Mischung aus 7-DAFSK und PVP in einem Gewichtsverhältnis von 2:1 bis 1:4 hergestellt, bevorzugt 2:1, 2:1.5, 1:1, 2:3, 1:2, 1:3 und 1:4, ganz besonders bevorzugt 1:1 oder 1:2.

Die Anwendung des erfindungsgemäßen Verfahrens erlaubt die effiziente und unkomplizierte Herstellung von 7-DAFSK-PVP Komplexen.

Das erfindungsgemäße Verfahren erlaubt die Herstellung von 7-DAFSK-PVP Komplexen mit einem hohen Masseanteil von 7-DAFSK. Herkömmliche Verfahren zur Herstellung von PVP Komplexen umfassen die Lösung der Komponenten in einem Lösungsmittel oder Lösungsmittelgemisch, wobei das Gemisch nicht über die Glasübergangstemperatur von PVP erhitzt wird. In einem hier erläuterten spezifischen Beispiel wurden mit dem erfindungsgemäßen Verfahren um 8-9 % höhere mittlere Masseanteile von 7-DAFSK in 7-DAFSK-PVP Komplexen im Vergleich zu herkömmlichen Komplexierungsverfahren erreicht.

Ein hoher Masseanteil von 7-DAFSK in 7-DAFSK-PVP Komplexen ist besonders für die pharmazeutische Anwendung in der Diagnose und/oder Therapie vorteilhaft. Durch einen hohen Masseanteil von 7-DAFSK am erfindungsgemäßen Komplex kann die Wasserlöslichkeit von 7-DAFSK erhöht werden, ohne dass damit eine unvorteilhaft große Menge an PVP benötigt wird. Formulierungen mit Forskolinderivaten können damit auch materialsparender als bisher zusammengestellt werden. Dadurch, dass große Mengen an Adjuvantien (PVP) und damit einhergehende Nebenwirkungen vermieden werden können, erleichtert und verbessert die vorliegende Erfindung die Anwendung von 7-DAFSK in der Diagnose und/oder Therapie.

Der erfindungsgemäße 7-DAFSK-PVP Komplex kann für jeden diätischen oder medizinischen Zweck verwendet werden, bei welchem Forskolin und/oder 7-DAFSK anwendbar ist.

Die vorliegende Erfindung betrifft daher auch eine pharmazeutische Zusammensetzung, welche den erfindungsgemäßen 7-DAFSK-PVP Komplex mit 7-DAFSK als Wirkstoff enthält.

Der Begriff "pharmazeutische Zusammensetzung" wird hier in Bezug auf den erfindungsgemäßen 7-DAFSK-PVP Komplex mit 7-DAFSK als Wirkstoff verwendet, optional in Verbindung mit weiteren Inhaltsstoffen und/oder Wirkstoffen.

Die erfindungsgemäße pharmazeutische Zusammensetzung kann in einer Reihe von systemischen und topischen Formulierungen vorliegen. Nicht-limitierende Beispiele von systemischen oder topischen Formulierungen enthaltend den erfindungsgemäßen 7-DAFSK-PVP Komplex umfassen orale, intrabukkale, intrapulmonale, rektale, intrauterinere, intradermale, topische, dermale, parenterale, intratumoröse, intrakranielle, bukkale, sublinguale, nasale, subkutane, intravaskuläre, intrathekale, inhalierbare, respirable, intraartikuläre, intrakavitäre, implantierbare, transdermale, ionotophoretische, intraokulare, vaginale, optische, intravenöse, intramuskuläre, intraglanduläre, intraorganische, intralymphatische Formulierungen, enterische Beschichtungen, oder Formulierungen mit langsamer Wirkstofffreisetzung.

Die Zusammensetzung kann einmal oder mehrmals täglich verabreicht werden. Eine bevorzugte Ausführungsform betrifft eine erfindungsgemäße pharmazeutische Zusammensetzung zur oralen, intravenösen oder inhalativen Verabreichung.

Formulierungen zur oralen Verabreichung können in diskreten Einheiten wie Kapseln, Tabletten, oder Lutschtabletten bereitgestellt werden, welche die Formulierung als Pulver oder Granulat enthalten. Formulierungen zur oralen Verabreichung können auch als Lösung oder Suspension in wässrigem oder nicht-wässrigem Medium, oder als Emulsion bereitgestellt werden.

Formulierungen zur intravenösen Verabreichung können als Lösung oder Suspension im wässrigen Medium bereitgestellt werden, z.B. als Salzlösung.

Die spezifische Dosierung des 7-DAFSK-PVP Komplexes zur Erzielung eines therapeutischen oder prophylaktischen Effektes hängt von der konkreten Anwendung ab, z.B. der Verabreichungsform, dem Alter, Gewicht und Zustand des individuellen Patienten, der behandelten Krankheit und der Stärke der Symptome.

Die Dosis an 7-DAFSK-PVP in der erfindungsgemäßen Zusammensetzung ist bevorzugt eine effektive Dosis, welche einen therapeutischen oder prophylaktischen Effekt erzielt, ohne unerwünschte Nebeneffekte zu verursachen, wobei die Zusammensetzung als Einzeldosis oder in mehreren Einheiten verabreicht werden kann.

In einer bestimmten Ausführungsform wird die erfindungsgemäße Zusammensetzung als Lösung oder Aerosol bereitgestellt, enthaltend 7-DAFSK in einer Konzentration von mindestens 25 mg/L enthält, vorzugsweise mindestens 50 mg/L, 75 mg/L, 100 mg/L, 200 mg/L, 300 mg/L, 400 mg/L, 500 mg/L, 600 mg/L, 800 mg/L oder 1000 mg/L. In einer bevorzugten Ausführungsform enthält die Zusammensetzung 7-DAFSK in einer Konzentration von mindestens 1 mg/mL, 1.5 mg/mL, 2 mg/mL, 2.5 mg/mL, 3 mg/mL, oder 4 mg/mL, besonders bevorzugt 4 mg/mL.

In einer weiteren bestimmten Ausführungsform wird die erfindungsgemäße Zusammensetzung als Kapsel oder Tablette bereitgestellt, enthaltend 7-DAFSK in einer pharmazeutisch wirksamen Menge, beispielsweise 10 mg bis 2000 mg, bevorzugt mindestens 25 mg, 50 mg, 100 mg, 200 mg, 300 mg, 400 mg, 500 mg, 600 mg, 700 mg, 800 mg, 900 mg oder 1000 mg, besonders bevorzugt 500 mg.

Forskolin und 7-DAFSK sind als unspezifische cAMP Stimulatoren bekannt. Der erfindungsgemäße 7-DAFSK-PVP Komplex kann zur Behandlung von jeder Krankheit oder Störung verwendet werden, welche mit Forskolin und/oder 7-DAFSK behandelt werden kann. Nicht-limitierende Beispiele solcher Krankheiten sind neurodegenerative Krankheiten, Alzheimer, motorische Fehlfunktionen, akute und chronische Herzkreislaufkrankheiten, Lungenkrankheiten wie Asthma, zystische Fibrose, vaskuläre Krankheiten im Zusammenhang mit zystischer Fibrose, Bronchitis, chronisch obstruktive Lungenerkrankung (COPD), Übergewicht, Glaukom, Gelenksentzündung, Arthrose/Arthritis, fibrotische Veränderungen wie idiopathische pulmonale Fibrose, posttraumatische pulmonale Fibrose, Bronchopulmonale Dysplasie (BPD), posttoxische Leberkrankheiten wie VPD oder Leberzirrhose, und periphere Durchblutungsstörungskrankheiten einschließlich Raynaud's Krankheit und Sklerodermie.

In einer speziellen Ausführungsform wird die erfindungsgemäße pharmazeutische Zusammensetzung zur Behandlung von Herz-Kreislauferkrankungen, Asthma bronchiale, Fettleibigkeit, Glaukom, Lungenerkrankungen, Gelenksentzündung, oder Arthrose/Arthritis verwendet.

Ein weiterer Aspekt der vorliegenden Erfindung betrifft eine erfindungsgemäße pharmazeutischen Zusammensetzung zur Verwendung als Medikament.

Die vorliegende Erfindung wird weiters durch die folgenden Beispiele und die Figuren illustriert, ohne darauf limitiert zu sein.

### BEISPIELE

Die folgenden Beispiele beschreiben die Synthese von 7-DAFSK und die Komplexierung von 7-DAFSK mit, wobei PVP mit einer mittleren molaren Masse von etwa 2,5 kD ("PVP K12") und etwa 24 kD ("PVP K25") gewählt wurde, und die Mengenverhältnisse von 7-DAFSK zu PVP variiert wurden. Die Beispiele behandeln weiters die Charakterisierung der erhaltenen Komplexe in Bezug auf den Gehalt an 7-DAFSK, die Wasserlöslichkeit, und die Wirkung als cAMP Aktivator in cAMP Assays. Die Beispiele enthalten keine detaillierten Beschreibungen von Standardmethoden, wie der Durchführung einer Gehaltsbestimmung mittels HPLC, oder der Messung von NMR Spektren. Solche Methoden sind der Fachperson gut bekannt.

### Beispiel 1 - Synthese von 7-Deacetylforskolin

Als Edukt für die Synthese von 7-Deacetyl Forskolin (7-DAFSK) wurde das synthetische Forskolin (GMP - Material) von Mercachem, Charge MAMA07-024-5, verwendet. Die Deacetylierung von Forskolin wurde über eine klassische Carbonatverseifung durchgeführt.

Die Deacetylierung erfolgte nach dem in Figur 1 abgebildeten Reaktionsschema. Zunächst wurden 200 mg Forskolin (synthetisches Forskolin, Mercachem, Charge MAMA07-024-5) und 200 mg calciniertes Na₂CO₃ in 10 mL Methanol im 250 mL Rundkolben, 4 Stunden bei Raumtemperatur gerührt. Anschließend wurde das Reaktionsgemisch über Nacht stehen gelassen (20 Stunden).

Danach wurde das Methanol abdestilliert (Rotavapor) und mit etwa 20 mL CH₂Cl₂ aus dem Kolben gelöst. Dabei wurde kurz mit Ultraschall behandelt (1 min). Das gebildete 7-DAFSK geht mit CH₂Cl₂ in Lösung, wobei die Salze ausfallen. Die Salze wurden abfiltriert (mittelschnelle Filter). Danach wurde das Filtrat mit 2 n HCl im Scheidetrichter gewaschen. Die Dichlormethan Fraktion wurde abgezogen und das Lösungsmittel im Rotavapor abdestilliert. Das so erhaltene weiße Pulver wurde mittels HPLC analysiert. Dabei konnte eine Reinheit von über 99 % bestimmt werden.

Die Struktur von 7-DAFSK wurde mittels ¹H NMR ¹³C NMR verifiziert. Die gemessenen ¹H und ¹³C NMR Spektren des erhaltenen 7-DAFSK sind ident zu bereits publizierten Spektren dieser Verbindung (J. Org. Chem. 2006, 71, 4619 - 4624). Damit konnte die gewünschte Deacetylierung von Forskolin nach dem in Figur 1 gezeigten Reaktionsschema bestätigt werden.

Das erhaltene 7-DAFSK wurde weiters mittels HPLC charakterisiert. Deacetylforskolin und Forskolin wurden mit einer mobilen Phase aus Acetonitril/Wasser (55/45 v/v) mit einer Flussrate von 0,6 mL/min eluiert (stationäre Phase: Nucleosil 120 3C18, 250 x 4 mm). Zur quantitativen Bestimmung des Gehaltes an 7-DAFSK im Komplex mit PVP wurde eine Kalibriergerade erstellt. Für die Kalibration wurden Kalibrierlösungen mit 7-DAFSK Konzentrationen von 0,014; 0,028; 0,056 und 0,56 mg/mL verwendet.

### Beispiel 2 - Herstellung von 7-DAFSK-PVP Komplexen

Da 7-Deacetylforskolin selbst nur mäßig wasserlöslich ist wurde 7-DAFSK mit PVP komplexiert, um die Wasserlöslichkeit zu steigern. Die 7-DAFSK-Komplexe wurde mit den erfindungsgemäßen Verfahren, im Folgenden "Erhitzungsverfahren" genannt, sowie zum Vergleich mit herkömmlicher Lösung ohne Erhitzung ("Lösungsverfahren") hergestellt.

### 1. Herstellung von 7-DAFSK-PVP Komplexen mittels Erhitzens von PVP über die Glasübergangstemperatur ("Erhitzungsverfahren")

### 7-DAFSK-PVP K12-Komplex (Komplex von 7-DAFSK mit PVP K12)

Für die Komplexierung von 7-Deacetylforskolin mit PVP K12 ("Kollidon" 12 PF, Ph. Eur., USP, JP; BASF, mittlere molare Masse: etwa 2,5 kD) wurden 20 mg 7-DAFSK und 20 mg PVP K12 in einem Porzellantiegel verrieben und mit 300 µL Ethanol versetzt und verrührt. Nach 10 min wurden 100 µL H₂O zugegeben, verrührt und wieder 10 min stehen gelassen. Anschließend wurde die Lösung im Trockenschrank innerhalb von 40 min auf 140°C erhitzt und insgesamt für 45 min darin belassen. Nach dem Abkühlen wurden 3 mL H₂O zugegeben und es wurde 20-30 min lang gerührt. Danach wurde die Suspension mit einer Spritze aufgenommen und durch 0,45 µm Filter (hydrophil) filtriert. Der Tiegel wurde mit 3 mL H₂O nachgewaschen und die Waschlösung ebenfalls durch den Filter filtriert. Der Gehalt an 7-DAFSK gelöst in den 6 mL H₂O wurde mittels HPLC bestimmt. Für die Bestimmung wurde die 7-DAFSK-PVP-Komplexlösung 1:10 mit Laufmittel verdünnt. Es wurde ein Masseanteil von 7-DAFSK am Gesamtkomplex von 35 Gew.-% bestimmt.

### 7-DAFSK-PVP K25-Komplex (Komplex von 7-DAFSK mit PVP K25)

Für die Komplexierung von 7-Deacetylforskolin mit PVP K25 (FLUKA 81399, mittlere molare Masse: etwa 24 kD) wurden 20 mg 7-DAFSK und 20 mg PVP K25 in einem Porzellantiegel verrieben und mit 300 µL Ethanol versetzt und verrührt. Nach 10 min wurden 100 µL H₂O zugegeben, verrührt und wieder 10 min stehen gelassen. Anschließend wurde die Lösung im Trockenschrank innerhalb von 40 min auf 185°C erhitzt und insgesamt für 45 min darin belassen. Nach dem Abkühlen wurden 3 mL H₂O zugegeben und 20-30 min lang gerührt. Danach wurde die Suspension mit einer Spritze aufgenommen und durch 0,45 µm Filter (hydrophil) filtriert. Der Tiegel wurde mit 3 mL H₂O nachgewaschen und die Waschlösung ebenfalls durch den Filter filtriert. Der Gehalt an 7-DAFSK gelöst in den 6 mL H₂O wurde mittels HPLC bestimmt. Für die Bestimmung wurde die 7-DAFSK-PVP 25-Komplexlösung 1:10 mit Laufmittel verdünnt. Es wurde ein Masseanteil von 7-DAFSK am Gesamtkomplex von 17 Gew.-% bestimmt.

### 2. Herstellung von 7-DAFSK-PVP Komplexen mittels Lösungsverfahren

### 7-DAFSK-PVP K12-Komplex

Es wurden 20 mg 7-DAFSK und 40 mg PVP K12 in einem Porzellantiegel verrieben und mit 300 µL Ethanol versetzt und verrührt. Nach 10 min wurden 100 µL H2O zugegeben, verrührt und wieder 10 min stehen gelassen. Anschließend wurde die Lösung bei Raumtemperatur für 45 min stehen gelassen, 3 mL H₂O zugegeben und 20-30 min lang gerührt. Danach wurde die Suspension mit einer Spritze aufgenommen und durch 0,45 µm Filter (hydrophil) filtriert. Der Tiegel wurde mit 3 mL H₂O nachgewaschen und die Waschlösung ebenfalls durch den Filter filtriert. Der Gehalt an 7-DAFSK gelöst in den 6 mL H₂O wurde mittels HPLC bestimmt. Es wurde ein Masseanteil von 7-DAFSK am Gesamtkomplex von 24 Gew.-% bestimmt.

### 7-DAFSK-PVP K25-Komplex

Es wurden 20 mg 7-DAFSK und 40 mg PVP 25 in einem Porzellantiegel verrieben und mit 300 µL Ethanol versetzt und verrührt. Nach 10 min wurden 100 µL H2O zugegeben, verrührt und wieder 10 min stehen gelassen. Anschließend wurde die Lösung bei Raumtemperatur für 45 min stehen gelassen, 3 mL H₂O zugegeben und für 20-30 min gerührt. Danach wurde die Suspension mit einer Spritze aufgenommen und durch 0,45 µm Filter (hydrophil) filtriert. Der Tiegel wurde mit 3 mL H₂O nachgewaschen und die Waschlösung ebenfalls durch den Filter filtriert. Der Gehalt an 7-DAFSK gelöst in den 6 mL H₂O wurde mittels HPLC bestimmt. Es wurde ein Masseanteil von 7-DAFSK am Gesamtkomplex von 8 Gew.-% bestimmt.

### Beispiel 3 - Bestimmung der Wasserlöslichkeit

Für die Löslichkeitstests wurde der entsprechende, mittels Rotationsverdampfer getrocknete, Komplex verwendet. Es wird eine definierte Menge Wasser vorgelegt und anschließend so lange Komplex zugegeben, bis ein Überschuss an 7-DAFSK-PVP vorliegt. Die übersättigte Lösung wird mit Ultraschall behandelt, gerührt und leicht erwärmt (Wasserbad 40°C). Anschließend wird die Suspension filtriert (0,45 µm Filter) und der Gehalt des Filtrats an 7-DAFSK mittels HPLC bestimmt.

### Beispiel 4 - Charakterisierung des 7-DAFSK-PVP Komplexes

### Masseanteil von 7-DAFSK am Gesamtkomplex

Figur 2 zeigt ein Diagramm des bestimmten Gehalts an 7-DAFSK im 7-DAFSK-PVP K12 und 7-DAFSK-PVP 25 Komplex, bei welchen ein Verhältnis von 7-DAFSK zu PVP von 20 zu 20 gewählt wurde. Hierbei wird zwischen dem Erhitzungsverfahren und dem Lösungsverfahren unterschieden. Bei allen Komplexen sind deutliche Unterschiede in Bezug auf die Gehälter an 7-DAFSK zwischen den beiden Methoden zu erkennen. Mit Hilfe des Erhitzungsverfahrens lassen sich um 8-9 % höhere Gehälter an 7-DAFSK in den fertigen Komplexen erzielen. Der 7-DAFSK-PVP K12 Komplex weist den höchsten Gehalt an 7-DAFSK auf (ca. 35 Gew.-%).

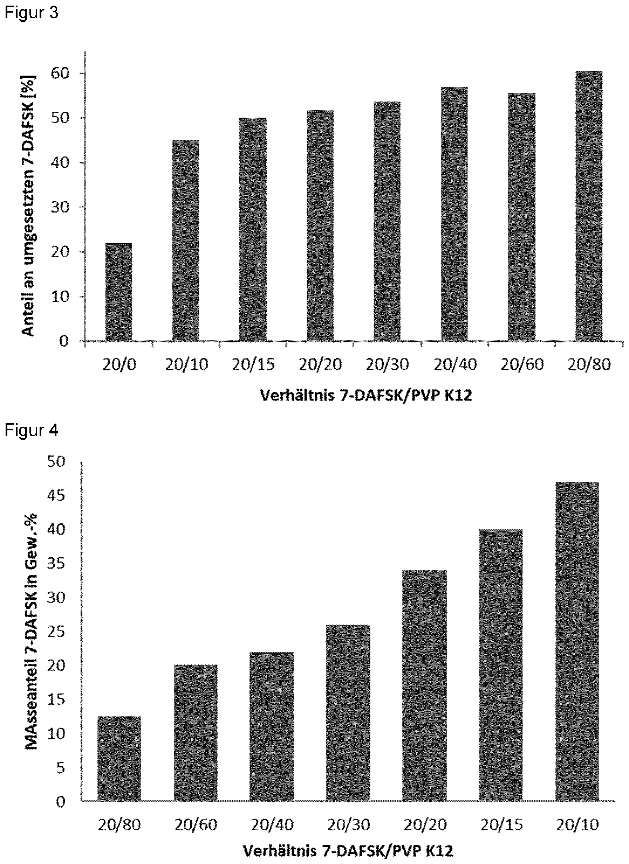

Zur Optimierung des Gehalts von 7-DAFSK am Gesamtkomplex wurden die eingesetzten Gewichtsverhältnisse von 7-DAFSK und PVP K12 variiert (Figur 3). Auch durch Variation der Verhältnisse an 7-DAFSK/PVP K12 konnten mit dieser Methode nicht mehr als 50-60% des eingesetzten Materials an 7-DAFSK (20 mg) komplexiert werden, wie aus Figur 3 ersichtlich wird. Es wird deutlich, dass sich trotz Erhöhung der Menge an PVP12 die schlussendlich komplexierte Menge an 7-DAFSK im fertigen Komplex nicht maßgeblich ändert. Um herauszufinden, wieviel nicht komplexiertes 7-DAFSK beim Lösevorgang nach der Herstellung mittels Erhitzungsverfahren in Lösung geht, wurde eine Kontrollprobe (ohne PVP12, nur 7-DAFSK), welche gleich wie die Komplex-Proben behandelt wurde, untersucht. Hierbei konnte weniger als ein Viertel des eingesetzten 7-DAFSK in Lösung gebracht werden. Die Komplexierung mit PVP bringt also eine Erhöhung der 7-DAFSK-Löslichkeit.

Auch durch Erhöhung der Lösungsmittelmenge vor dem Erhitzen konnten keine größeren Mengen an 7-DAFSK in den Komplex mit PVP K12 gebracht werden.

In Figur 4 ist zu sehen, dass auch mit sehr geringen Mengen an PVP vergleichsmäßige Ergebnisse erzielt werden konnten.

Die angegebenen Gehälter an 7-DAFSK in Gew.-% wurden mittels HPLC bestimmt. Für die Berechnung wurde angenommen, dass die gesamte Menge an PVP beim Lösen mit den 6 mL H₂O in Lösung geht.

### Wasserlöslichkeiten

Tabelle 2 zeigt, dass eine deutliche Steigerung der Wasserlöslichkeit von 7-DAFSK durch die Komplexierung mit PVP möglich ist. Die Löslichkeit von 7-DAFSK im Komplex mit PVP ist bis zu dreimal so hoch wie die Löslichkeit von nicht kompliziertem 7-DAFSK. Im Unterschied zu 7-DAFSK kann Forskolin aufgrund seiner sterischen Struktur mit PVP kaum gelöst werden, noch mit PVP komplexieren.

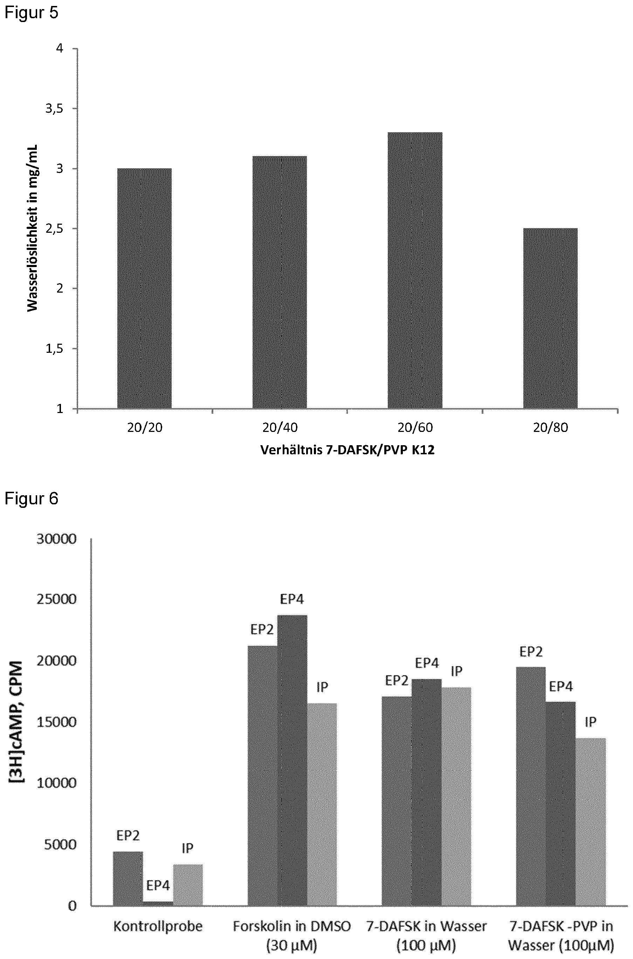

Figur 5 zeigt, dass sich die Wasserlöslichkeit bei unterschiedlichen Verhältnissen an DAFSK/PVP nicht ausschlaggeben ändert. Die Werte schwanken zwischen 2,5 mg/mL und 3,5 mg/mL. Dies liegt daran, dass bei jedem Verhältnis ungefähr derselbe Anteil an 7-DAFSK (50-60%) des komplexiert wird, wodurch sich die Wasserlöslichkeit des Komplexes nicht wesentlich ändert.

**Tabelle 2: Wasserlöslichkeiten unterschiedlicher Substanzen/Komplexe**

| **Substanz/Komplex** | **PVP Masseanteil am Gesamtkomplex** | **Wasserlöslichkeit** |
|---|---|---|
| FSK | - | 0,01 mg/mL |
| FSK-PVP12 | - | 0,001 mg/ml (nicht detektierbar, keine nachweisbare Komplexbildung mit Forskolin) |
| 7-DAFSK | - | 1 mg/mL |
| 7-DAFSK-PVP K12 | 35 Gew.-% | 3 mg/mL |
| 7-DAFSK-PVP K25 | 17 Gew.-% | 2 mg/mL |

### Beispiel 5 - Zellkulturtest: cAMP Assay

Ein cAMP Assay wurde mit HEK293 Zellen (humane embryonale Nierenzellen, 1970) mit den Prostaglandin Rezeptoren EP2 oder EP4, oder dem Prostacyclin Rezeptor IP, durchgeführt. Die HEK293 Zellen wurden mit FCS und einem Radioligand ([3H] Adenin) inkubiert. Die Bildung von cAMP ([³H]cAMP) wurde experimentell verfolgt.

Die Aktivierung der Adenylycyclase (AC) durch Forskolin (in DMSO gelöst), 7-DAFSK und 7-DAFSK-PVP, beide in Wasser gelöst, wurde verglichen. Es bestehen geringfügige Unterschiede der drei Forskolin Abkömmlinge bei der Aktivierung der AC über EP2 und EP4 Rezeptoren, und keine signifikanten Unterschiede bei der Aktivierung des IP Rezeptors (Figur 6).

Alle drei Forskolin Abkömmlinge aktivieren die Adenylylcyclase über die genannten Rezeptoren, auch in Anwesenheit von Treprostinil. Treprostinil alleine (Kontrollprobe) zeigt keine Aktivierung der AC. Die verbesserte Aktivität von Forskolin, 7-DAFSK und 7-DAFSK-PVP gegenüber der Kontrolle ist in Figur 6 deutlich erkennbar. Diese Ergebnisse zeigen somit, dass 7-DAFSK-PVP ein geeigneter Stimulator der Adenylylcyclase ist.

Die Assays wurden wie folgt durchgeführt:
Tag 1: HEK293 Zellen welche entweder EP2, EP4 oder IP Rezeptoren stabil exprimieren, wurden auf einer 6-Well Platte in 2 mL DMEM Medium mit 10% FCS and 2µCi [³H]-Adenin angesetzt und für 16 Stunden inkubiert.
Tag 2: Die Zellen wurden mit 30 µM Treprostinil alleine oder in Kombination mit 30 µm Forskolin in DMSO, 100 µM 7-DAFSK in Wasser oder 100 µM 7-DAFSK im 7-DAFSK-PVP K12 Komplex in Wasser stimuliert (in frischem Medium für 30 Minuten bei Raumtemperatur). Danach wurden die Zellen mit 2,5% Perchlorsäure (PCA) für 30 Minuten auf Eis lysiert. Das PCA Extrakt wurde mit KOH neutralisiert und [³H]cAMP wurde durch sequenzielle Chromatographie mit DOWEX und Aluminiumoxid Säulen getrennt. Die Radioaktivität wurde anschließend mit einem Szintillator gemessen.

Ein Aliquot des PCA Extraktes von jeder Well wurde für die Messung der Radioaktivität verwendet.

### Beispiel 6 - Herstellungsversuch eines Forskolin-PVP Komplexes mittels Lösungsverfahren

Es wurden 20 mg FSK und 40 mg PVP 25 in einem Porzellantiegel verrieben und mit 300 µL Ethanol (alternativ Propanol) versetzt und verrührt. Nach 10 min wurden 100 µL H2O zugegeben, verrührt und wieder 10 min stehen gelassen. Anschließend wurde die Lösung bei Raumtemperatur für 45 min stehen gelassen, 3 mL H₂O zugegeben und für 20-30 min gerührt. Danach wurde die Suspension mit einer Spritze aufgenommen und durch 0,45 µm Filter (hydrophil) filtriert. Der Tiegel wurde mit 3 mL H₂O nachgewaschen und die Waschlösung ebenfalls durch den Filter filtriert. Der Gehalt an FSK gelöst in den 6 mL H₂O wurde mittels HPLC bestimmt. Es wurde ein Masseanteil von FSK am Gesamtkomplex von <0,01 Gew.-% bestimmt. FSK war im Filtrat mittels HPLC nicht nachweisbar.

## Patentansprüche

1. Komplex aus 7-Deacetylforskolin (7-DAFSK) und Polyvinylpyrrolidon (PVP).

2. Komplex nach Anspruch 1, **dadurch gekennzeichnet, dass** PVP eine mittlere molare Masse bezogen auf das Massenmittel der Molmasse von 1 bis 40 kD hat, vorzugsweise von 2 bis 25 kD, besonders bevorzugt etwa 2,5 kD.

3. Komplex nach Anspruch 1 oder 2, **dadurch gekennzeichnet, dass** 7-DAFSK von synthetischem Forskolin stammt.

4. Komplex nach einem der Ansprüche 1 bis 3, **dadurch gekennzeichnet, dass** der mittlere Masseanteil von 7-DAFSK am Gesamtkomplex im Bereich von 1 bis 50 Gew.-% liegt, bevorzugt bei 30 Gew.-% oder höher.

5. Verfahren zur Herstellung eines Komplexes nach einem der Ansprüche 1 bis 4, **dadurch gekennzeichnet, dass** ein Gemisch von 7-DAFSK und PVP auf eine Temperatur erhitzt wird, die über der Glasübergangstemperatur des eingesetzten PVP liegt.

6. Verfahren nach Anspruch 5, **dadurch gekennzeichnet, dass** dem Gemisch von 7-DAFSK und PVP ein Lösungsmittel, vorzugsweise Wasser, Ethanol, Methanol, Aceton, Ethylmethylketon, Dichlormethan und/oder Ethylacetat, noch mehr bevorzugt Wasser, Ethanol, Methanol, Propanol und/oder Dichlormethan, noch mehr bevorzugt Wasser und/oder Ethanol, oder ein Gemisch, davon zugegeben wird.

7. Verfahren nach Anspruch 5 oder 6, **dadurch gekennzeichnet, dass** die Mischung für mindestens 5 Minuten auf einer Temperatur, die über der Glasübergangstemperatur des eingesetzten PVP liegt, gehalten wird.

8. Verfahren nach einem der Ansprüche 5 bis 7 enthaltend die Schritte:
a) Herstellung einer Mischung aus 7-DAFSK und PVP und Versetzen der Mischung mit einem Lösungsmittel, bevorzugt mit einem Alkohol, bevorzugt Ethanol,
b) Zugabe von Wasser oder einer wässrigen Lösung,
c) Erhitzen der Mischung über die Glasübergangstemperatur des PVPs für mindestens 5 Minuten,
d) Abkühlen der Mischung und Lösen in Wasser oder einer wässrigen Lösung,
e) Entfernen der ungelösten Bestandteile, optional durch Filtern.

9. Pharmazeutische Zusammensetzung enthaltend einen Komplex nach einem der Ansprüche 1 bis 4.

10. Pharmazeutische Zusammensetzung nach Anspruch 9 zur Verwendung als Medikament.

11. Pharmazeutische Zusammensetzung nach Anspruch 9, **dadurch gekennzeichnet, dass** die Zusammensetzung 7-DAFSK in einer Konzentration von mindestens 25 mg/L, bevorzugt mindestens 100 mg/mL, 250 mg/mL, 500 mg/mL, 1000 mg/mL, 2000 mg/mL, 3000 mg/mL oder 4000 mg/mL enthält, besonders bevorzugt 4000 mg/mL.

12. Pharmazeutische Zusammensetzung nach Anspruch 9 oder 11 zur intravenösen, oralen oder inhalativen Verabreichung.

13. Pharmazeutische Zusammensetzung nach einem der Ansprüche 9 bis 12 zur Verwendung in der Behandlung von Herz-Kreislauferkrankungen, Asthma bronchiale, Fettleibigkeit, Glaukom, Lungenerkrankungen, Gelenksentzündung, oder Arthrose/Arthritis.

## Claims

1. A complex of 7-deacetylforskolin (7-DAFSK) and polyvinylpyrrolidone (PVP).

2. The complex according to claim 1, **characterised in that** PVP has an average molar mass based on the mass average of the molar mass of 1 to 40 kD, preferably of 2 to 25 kD, particularly preferably of approximately 2.5 kD.

3. The complex according to claim 1 or 2, **characterised in that** 7-DAFSK originates from synthetic forskolin.

4. The complex according to one of claims 1 to 3, **characterised in that** the average mass percentage of 7-DAFSK in the overall complex is in the range of 1 to 50 wt%, preferably 30 wt% or higher.

5. A process for preparing a complex according to one of claims 1 to 4, **characterised in that** a mixture of 7-DAFSK and PVP is heated to a temperature above the glass transition temperature of the PVP used.

6. The process according to claim 5, **characterised in that** a solvent, preferably water, ethanol, methanol, acetone, ethyl methyl ketone, dichloromethane and/or ethyl acetate, even more preferably water, ethanol, methanol, propanol and/or dichloromethane, even more preferably water and/or ethanol, or a mixture thereof, is added to the mixture of 7-DAFSK and PVP.

7. The process according to claim 5 or 6, **characterised in that** the mixture is held at a temperature above the glass transition temperature of the PVP used for at least 5 minutes.

8. The process according to one of claims 5 to 7, containing the steps of:
a) preparing a mixture of 7-DAFSK and PVP and adding a solvent, preferably an alcohol, preferably ethanol, to the mixture,
b) adding water or an aqueous solution,
c) heating the mixture to above the glass transition temperature of the PVP for at least 5 minutes,
d) cooling the mixture and dissolving it in water or an aqueous solution,
e) removing the undissolved constituents, optionally by filtering.

9. A pharmaceutical composition containing a complex according to one of claims 1 to 4.

10. The pharmaceutical composition according to claim 9 for use as a medicament.

11. The pharmaceutical composition according to claim 9, **characterised in that** the composition contains 7-DAFSK in a concentration of at least 25 mg/l, preferably at least 100 mg/ml, 250 mg/ml, 500 mg/ml, 1000 mg/ml, 2000 mg/ml, 3000 mg/ml or 4000 mg/ml, particularly preferably 4000 mg/ml.

12. The pharmaceutical composition according to claim 9 or 11 for intravenous, oral or inhaled administration.

13. The pharmaceutical composition according to one of claims 9 to 12 for use in the treatment of cardiovascular diseases, bronchial asthma, obesity, glaucoma, lung diseases, inflammation of the joints, or arthritis/osteoarthritis.

## Revendications

1. Complexe formé de 7-déacétylforskoline (7-DAFSK) et de polyvinylpyrrolidone (PVP).

2. Complexe selon la revendication 1, **caractérisé en ce que** la PVP a une masse molaire moyenne de 1 à 40 kD, préférablement de 2 à 25 kD, de manière particulièrement préférable d'environ 2,5 kD par rapport à la masse moléculaire pondérée de la masse molaire.

3. Complexe selon la revendication 1 ou 2, **caractérisé en ce que** la 7-DAFSK est issue d'une forskoline synthétique.

4. Complexe selon l'une quelconque des revendications 1 à 3, **caractérisé en ce que** la part massique moyenne de la 7-DAFSK sur le complexe total se situe sur la plage de 1 à 50 % en poids, de préférence à 30 % en poids ou plus.

5. Procédé de fabrication d'un complexe selon l'une quelconque des revendications 1 à 4, **caractérisé en ce qu'**un mélange de 7-DAFSK et de PVP est chauffé à une température supérieure à la température de transition vitreuse de la PVP utilisée.

6. Procédé selon la revendication 5, **caractérisé en ce qu'**il est ajouté au mélange de 7-DAFSK et de PVP un solvant, préférablement de l'eau, de l'éthanol, du méthanol, de l'acétone, de l'éthylméthylcétone, du dichlorométhane et/ou de l'éthylacétate, encore plus préférablement de l'eau, de l'éthanol, du méthanol, du propanol et/ou du dichlorométhane, encore plus préférablement de l'eau et/ou de l'éthanol, ou un mélange de ceux-ci.

7. Procédé selon la revendication 5 ou 6, **caractérisé en ce que** le mélange est maintenu pendant au moins 5 minutes à une température supérieure à la température de transition vitreuse de la PVP utilisée.

8. Procédé selon l'une quelconque des revendications 5 à 7 comprenant les étapes de :
a) la fabrication d'un mélange formé de 7-DAFSK et de PVP et le mélange au mélange d'un solvant, préférablement d'un alcool, préférablement de l'éthanol ;
b) l'ajout d'eau ou d'une solution aqueuse ;
c) le chauffage du mélange au-delà de la température de transition vitreuse de la PVP pendant au moins 5 minutes ;
d) le refroidissement du mélange et la dissolution dans de l'eau ou dans une solution aqueuse ;
e) le retrait des composants non dissous, en option par filtrage.

9. Composition pharmaceutique contenant un complexe selon l'une quelconque des revendications 1 à 4.

10. Composition pharmaceutique selon la revendication 9 pour utilisation comme médicament.

11. Composition pharmaceutique selon la revendication 9, **caractérisée en ce que** la composition contient de la 7-DAFSK dans une concentration d'au moins 25 mg/l, préférablement d'au moins 100 mg/ml, 250 mg/ml, 500 mg/ml, 1000 mg/ml, 2000 mg/ml, 3000 mg/ml ou 4000 mg/ml, de manière particulièrement préférable de 4000 mg/ml.

12. Composition pharmaceutique selon la revendication 9 ou 11 pour une administration par intraveineuse, par voie orale ou par inhalation.

13. Composition pharmaceutique selon l'une quelconque des revendications 9 à 12 pour utilisation dans le traitement des maladies cardio-vasculaires, l'asthme bronchique, l'obésité, le glaucome, les maladies pulmonaires, l'inflammation des articulations, ou l'arthrose/l'arthrite.
